# FASCICULE DE BREVET EUROPEEN

(11) **EP 2 429 990 B1**
(45) Date de publication et mention de la délivrance du brevet: **23.01.2013**
(21) Numéro de dépôt: 10718143.0
(22) Date de dépôt: 29.04.2010
(51) Int. Cl.: C07C 313/04

(54) **PROCEDE DE PREPARATION DES ESTERS D'ACIDES FLUOROALCANESULFINIQUES**
VERFAHREN ZUR HERSTELLUNG VON FLUORALKANSULFINSÄUREESTERN
METHOD FOR PREPARING FLUOROALKANESULPHINIC ACID ESTERS

(30) Priorité: 04.05.2009 FR 0902136
(43) Date de publication de la demande: 21.03.2012
(73) Titulaire: Rhodia Opérations, 93306 Aubervilliers (FR)
(72) Inventeur: BUISINE, Olivier, F-69230 Saint-Genis Laval (FR)
(74) Mandataire: Blanchard, Isabelle Jackie
(86) Numéro de dépôt international: PCT/EP2010/055820
(87) Numéro de publication internationale: WO 2010/127991

(56) Documents cités:
- D.T. SAUER, ET AL.: "Chemistry of trifluoromethyl sulphinyl fluoride. Trifluoromethylsulphinamides and trifluoromethylsulphinate esters" INORGANIC CHEMISTRY, vol. 10, no. 2, février 1971 (1971-02), pages 358-362, XP002559952 AMERICAN CHEMICAL SOCIETY, WASHINGTON, DC, US ISSN: 0020-1669 DOI: 10.1021/ic50096a028 cité dans la demande
- J.B. HENDRICKSON, ET AL.: "Synthetic manipulation of the triflone group. Formation from alcohols, constructions, and conversion to ketones and amines" TETRAHEDRON, vol. 32, no. 14, juillet 1976 (1976-07), pages 1627-1635, XP002559953 ELSEVIER SCIENCE PUBLISHERS, AMSTERDAM, NL ISSN: 0040-4020 DOI: 10.1016/0040-4020(76)85149-6 cité dans la demande
- T. BILLARD, ET AL.: "A new equivalent of the CF3S(O)+ cation. Synthesis of trifluoromethanesulphinates and trifluoromethanesulphinamides" TETRAHEDRON, vol. 55, no. 23, 4 juin 1999 (1999-06-04), pages 7243-7250, XP002559954 ELSEVIER SCIENCE PUBLISHERS, AMSTERDAM, NL ISSN: 0040-4020 DOI: 10.1016/S0040-4020(99)00364-6 cité dans la demande
- M. HANACK, ET AL.: "1-Phenyl-3-(trifluormethansulfonyl)propad ien" TETRAHEDRON LETTERS, vol. 22, no. 6, 1981, pages 557-558, XP002559957 ELSEVIER SCIENCE PUBLISHERS, AMSTERDAM, NL ISSN: 0040-4039 DOI: 10.1016/S0040-4039(01)90153-4
- C. HARZDORF, ET AL.: "Über Perfluoralkansulfinsäuren" LIEBIGS ANNALEN DER CHEMIE, no. 1, 22 février 1973 (1973-02-22), pages 33-39, XP002559956 VERLAG CHEMIE, WEINHEIM, DE ISSN: 0170-2041 DOI: 10.1002/jlac.197319730106

## Description

La présente invention a pour objet un procédé de préparation des esters d'acides fluoroalcanesulfiniques.

L'invention vise plus particulièrement la préparation des esters de l'acide trifluorométhanesulfinique dénommé couramment « acide triflinique ».

Les esters d'alkyle ou aromatiques de l'acide triflinique souvent désignés par le terme « triflinate » sont des produits décrits dans la littérature.

Il a été proposé différents procédés pour la synthèse desdits esters.

L'un consiste à faire réagir un alcool avec une forme activée de l'acide triflinique (chlorure, fluorure).

Ainsi Sauer Dennis T. et Shreeve Jeanne M. [Inorganic Chemistry (1971), 10(2), 358 - 362] ont décrit l'addition du méthanol sur le fluorure de trifluorométhanesulfinyle.

De même, l'addition de l'éthanol sur le chlorure de triflinyle est décrite en milieu basique par Hendrickson James B. et Skipper Paul L. [Tetrahedron (1976), 32 (14), 1627 - 35].

Dans ces deux exemples, le motif trifluorométhanesulfinyle joue le rôle d'électrophile et celui-ci peut être généré *in situ.*

Billard T., Greiner A. et Langlois B. [Tetrahedron (1999), 55, 7243-50] ont décrit l'activation du triflinate de sodium par l'oxychlorure de phosphore dans l'acétate d'éthyle. L'addition de phénol conduit à l'ester phénylique de l'acide triflinique F₃C - SO - OPh avec un rendement de 73%.

Une autre voie d'accès est fondée sur le principe de l'attaque nucléophile d'un ion triflinate sur un agent alkylant.

La réaction du triflinate de potassium et du chlorure d'éthyle [Hendrickson James B., Giga Aziz, Wareing James, J. Amer. Chem. Soc. (1974), 96, 2275] ne conduit pas au triflinate d'éthyle F₃C - SO - OC₂H₅ mais principalement au produit de S-alkylation c'est-à-dire à une sulfone de formule F₃C - SOO - C₂H₅.

Pour accéder à un triflinate d'alkyle, il a été proposé [Hendrickson, James et al, loc. cit.] de faire réagir le triflinate de potassium avec le p-nitrobenzènesulfonate d'isopropyle. Outre l'utilisation d'un réactif peu courant, le rendement obtenu en triflinate d'isopropyle varie entre 23 et 66%.

La difficulté d'obtenir un triflinate d'alkyle réside aussi dans le fait que les triflinates d'alkyle ont la faculté de s'isomériser en sulfone [Hendrickson, James et al, loc. cit.] comme l'illustre la réaction suivante :

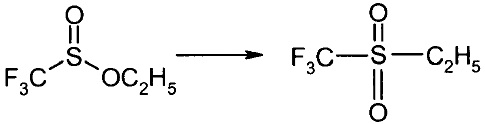

La Demanderesse se propose de fournir un procédé permettant d'obtenir les esters d'acide triflinique tout en évitant les inconvénients précités.

Il a maintenant été trouvé et c'est ce qui constitue l'objet de la présente invention, un procédé de préparation d'un ester d'un acide fluoroalcanesulfinique caractérisé par le fait qu'il comprend la réaction d'un acide fluoroalcanesulfinique avec un carbonate organique conduisant à la formation d'un ester de l'acide fluoroalcanesulfinique et de dioxyde de carbone qui est éliminé au cours de la réaction.

Selon un mode de réalisation préféré du procédé de l'invention, on réalise la préparation d'un ester de l'acide triflinique par réaction de l'acide triflinique et d'un carbonate de dialkyle.

On donne ci-après, le schéma réactionnel du procédé de l'invention pour faciliter la compréhension de l'invention sans pour autant lier la portée de l'invention, à celui-ci : (R représentant un groupe alkyle).

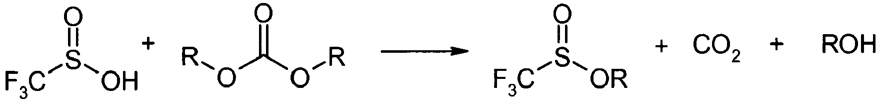

Conformément au procédé de l'invention, on effectue une réaction de trans-estérification conduisant à l'obtention d'un triflinate d'alkyle, de dioxyde de carbone et d'un alcool provenant du carbonate organique.

Selon une variante préférée du procédé de l'invention, l'élimination du dioxyde carbone au fur et à mesure de sa formation, favorise l'obtention de l'ester de l'acide fluoroalcanesulfinique.

Le procédé de l'invention s'applique plus particulièrement aux acides fluoroalcanesulfiniques répondant à la formule suivante : 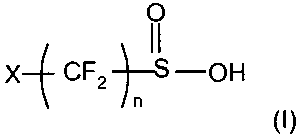 dans ladite formule :
- X représente un atome d'hydrogène ou un atome de fluor,
- n représente un nombre entre 1 et 8.

L'invention vise plus particulièrement les acides perfluoroalcanesulfiniques répondant à la formule (I) dans laquelle X est un atome de fluor.

Dans la formule (I), x varie entre 1 et 8 mais est égal de préférence à 1.

Comme exemples préférés d'acides fluoroalcanesulfiniques, on peut citer
- l'acide difluorométhanesulfinique,
- l'acide trifluorométhanesulfinique,
- l'acide perfluorobutanesulfinique,
- l'acide perfluorooctanesulfinique.

L'invention convient tout à fait bien à la préparation des esters de l'acide trifluorométhanesulfinique ou acide triflinique.

Conformément au procédé de l'invention, on fait réagir l'acide fluoroalcanesulfinique avec un carbonate organique.

Les carbonates organiques intervenant dans le procédé de l'invention répondent plus particulièrement à la formule générale suivante :

R₁-O-CO-O-R₂ (II)

dans ladite formule :
- R₁ représente :
   - un groupe alkyle, linéaire ou ramifié, ayant 1 à 6 atomes de carbone,
   - un groupe cycloalkyle ayant 5 à 6 atomes de carbone,
   - un groupe cycloalkyle, ayant 5 ou 6 atomes de carbone substitué par un à trois groupes alkyle ayant 1 à 4 atomes de carbone et/ou par un ou deux atomes d'halogène,
   - un groupe phényle,
   - un groupe phényle substitué par un à trois groupes alkyles ayant 1 à 4 atomes de carbone et/ou par un ou deux atomes d'halogène,
- R₂ représente :
   - un groupe alkyle, linéaire ou ramifié, ayant 1 à 6 atomes de carbone,
   - un groupe cycloalkyle ayant 5 à 6 atomes de carbone,
   - un groupe cycloalkyle, ayant 5 ou 6 atomes de carbone substitué par un à trois groupes alkyle ayant 1 à 4 atomes de carbone et/ou par un ou deux atomes d'halogène,
   - un groupe phényle,
   - un groupe phényle substitué par un à trois groupes alkyle ayant 1 à 4 atomes de carbone et/ou par un ou deux atomes d'halogène,
- R₁ et R₂ peuvent former ensemble un groupe alkylène ayant 2 à 6 atomes de carbone.

Bien que R₁ puisse être différent de R₂, il est souhaitable par un souci de simplification que R₁ soit identique à R₂.

Comme exemples de groupes R₁, R₂, on peut citer les groupes alkyle ayant de 1 à 4 atomes de carbone, de préférence méthyle, éthyle, isopropyle ; le groupe cyclohexyle ; le groupe phényle : un groupe phényle substitué en ortho et ortho' par un atome d'halogène de préférence chlore ou brome ou par un groupe alkyle linéaire ou ramifié ayant de 1 à 4 atomes de carbone.

Dans la formule (II), R₁ et R₂ peuvent former un groupe alkylène, de préférence un groupe éthylène ou propylène.

Comme exemples de carbonates organiques, on peut citer : le carbonate de diméthyle, le carbonate de diéthyle, le carbonate de diisopropyle, le carbonate de phényle et de tertiobutyle, le carbonate d'éthylène, le carbonate de propylène.

Dans la liste précitée, le carbonate de diméthyle ou de diéthyle sont choisis préférentiellement.

La quantité de carbonate organique mise en oeuvre exprimée par rapport à l'acide fluoroalcanesulfinique est généralement au moins égale à la quantité stoechiométrique.

Ainsi, le rapport entre le nombre de moles de carbonate organique et le nombre de moles d'acide fluoroalcanesulfinique varie avantageusement entre 1 et 2 et se situe de préférence entre 1 et 1,2.

La présence d'eau dans le milieu réactionnel influe sur le rendement réactionnel. Ainsi, il est préférable que le procédé de l'invention soit conduit dans des conditions anhydres. Il y a lieu de veiller à ce que les réactifs soient anhydres. Une quantité d'eau allant jusqu'à environ 1 % en masse dans le milieu peut être tolérée.

La température de la réaction est choisie de manière qu'elle soit suffisante pour permettre l'accomplissement de la réaction trans-estérification et d'empêcher la réaction d'isomérisation compétitive.

La température de la réaction est choisie de préférence entre 0 et 100°C, et de préférence entre 60 et 95°C.

La réaction est conduite avantageusement sous pression atmosphérique.

Des pressions légèrement inférieures ou supérieures peuvent être également utilisées.

On conduit la réaction de préférence sous atmosphère d'un gaz inerte qui peut être l'azote ou un gaz rare, de préférence l'argon : l'azote étant préféré notamment compte tenu de son coût réduit.

D'un point de vue pratique, le procédé selon l'invention est simple à mettre en oeuvre.

Les différents réactifs peuvent être introduits dans n'importe quel ordre.

D'une manière préférée, on préfère introduire progressivement l'acide par fractions ou en continu sur le carbonate organique.

On porte le milieu réactionnel à la température désirée, tout en maintenant le milieu réactionnel sous agitation.

Au cours de la réaction, il y a formation de dioxyde de carbone qui est éliminé au cours de la réaction.

Selon un mode de réalisation préféré de l'invention, le dioxyde de carbone est éliminé au fur et à mesure de sa formation.

Le dioxyde de carbone libéré peut être éventuellement piégé par une solution basique par exemple par introduction dans une colonne d'abattage à la soude ou à la potasse.

La durée de la réaction varie entre 2 et 20 heures, de préférence, entre 5 et 10 heures.

En fin de réaction, on obtient l'ester de l'acide fluoroalcanesulfinique et l'alcool qui correspond au carbonate organique de départ.

L'ester de l'acide fluoroalcanesulfinique est récupéré à partir de ce milieu selon les techniques classiques de séparation notamment par distillation de préférence sous une pression réduite variant par exemple entre 5 et 200 mbar ou bien par cristallisation.

Le procédé de l'invention est avantageusement conduit dans un appareillage susceptible de résister à la corrosion du milieu réactionnel.

A cet effet, on choisit des matériaux pour la partie en contact avec le milieu réactionnel résistant à la corrosion comme les alliages à base de molybdène, chrome, cobalt, fer, cuivre manganèse, titane, zirconium, aluminium, carbone et tungstène vendus sous les marques HASTELLOY^{®} ou les alliages de nickel, chrome, fer, manganèse additivés de cuivre et/ou molybdène commercialisés sous la dénomination INCONEL^{®} et plus particulièrement les alliages HASTELLOY C 276 ou INCONEL 600, 625 ou 718.

On peut choisir également les aciers inoxydables, tels que les aciers austénitiques [Robert H. Perry et al, Perry's Chemical Engineers' Handbook, Sixth Edition (1984), page 23-44]. et plus particulièrement les aciers inoxydables 304, 304 L, 316 ou 316 L. On met en oeuvre un acier ayant une teneur en nickel au plus de 22% en masse, de préférence comprise entre 6 et 20%, et plus préférentiellement comprise entre 8 et 14%.

Les aciers 304 et 304 L ont une teneur en nickel variant entre 8 et 12% et les aciers 316 et 316 L ont une teneur en nickel variant entre 10 et 14%. On fait appel plus particulièrement aux aciers 316 L.

On peut aussi faire appel aux aciers vitrifiés avec addition éventuelle d'inhibiteurs de corrosion comme par exemple la silice ou l'acide borique.

L'ensemble des différentes étapes du procédé de l'invention peuvent être mises en oeuvre en continu ou en discontinu.

Le procédé de l'invention est particulièrement intéressant car il présente de nombreux avantages.

Il s'agit d'un procédé simple et économique qui ne conduit pas à la formation de sulfone.

On donne ci-après des exemples de réalisation de l'invention. Ces exemples sont donnés à titre illustratif et sans caractère limitatif.

Dans les exemples, on définit le taux de conversion et le rendement obtenu.

Le taux de conversion (TT) correspond au rapport entre le nombre de substrat (acide trifluorométhanesulfinique) transformées et le nombre de moles de substrat (acide trifluorométhanesulfinique) engagées.

Le rendement (RR) correspond au rapport entre le nombre de moles de produit formées (ester de l'acide trifluorométhanesulfinique) et le nombre de moles de substrat (acide trifluorométhanesulfinique) engagées.

### Exemple

Dans un réacteur en verre de 20mL, on charge 13,4 g d'acide trifluorométhanesulfinique (0,1 mol).

On ajoute 5,9 g de carbonate de diéthyle (0,05 mol) et l'on porte le mélange à 90°C pendant 10 heures.

L'ensemble est en fin de réaction ramené à la température ambiante (20°C).

L'analyse RMN¹⁹F du milieu réactionnel brut indique un taux de transformation de 49% de l'acide triflinique et un rendement de 49% en triflinate d'éthyle.

Le réacteur est surmonté d'une colonne vigreux et l'ensemble est porté à une température de 58°C sous une pression de 175 mbar.

On recueille une fraction de distillation (9 g) à 38°C qui est une fraction de liquide incolore contenant 89% en masse de trifluorométhanesulfinate d'éthyle.

## Revendications

1. Procédé de préparation d'un ester d'un acide fluoroalcanesulfinique **caractérisé par le fait qu'**il comprend la réaction d'un acide fluoroalcanesulfinique avec un carbonate organique conduisant à la formation d'un ester de l'acide fluoroalcanesulfinique et de dioxyde de carbone qui est éliminé au cours de la réaction.

2. Procédé selon la revendication 1 **caractérisé par le fait que** l'acide fluoroalcanesulfinique répond à la formule suivante : 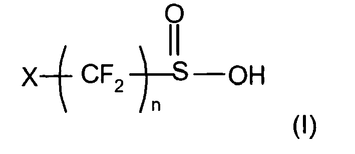 dans ladite formule :
- X représente un atome d'hydrogène ou un atome de fluor,
- n représente un nombre entre 1 et 8.

3. Procédé selon la revendication 2 **caractérisé par le fait que** l'acide fluoroalcanesulfinique est l'acide difluorométhanesulfinique, un acide perfluoroalcanesulfinique de préférence l'acide trifluorométhanesulfinique, l'acide perfluorobutanesulfinique, l'acide perfluorooctanesulfinique.

4. Procédé selon la revendication 2 **caractérisé par le fait que** l'acide fluoroalcanesulfinique est l'acide trifluorométhanesulfinique.

5. Procédé selon l'un des revendications 1 à 4 **caractérisé par le fait que** le carbonate organique répond à la formule générale suivante :
R₁-O-CO-O-R₂ (II)
dans ladite formule :
- R₁ représente :
- un groupe alkyle, linéaire ou ramifié, ayant 1 à 6 atomes de carbone,
- un groupe cycloalkyle ayant 5 à 6 atomes de carbone,
- un groupe cycloalkyle, ayant 5 ou 6 atomes de carbone substitué par un à trois groupes alkyle ayant 1 à 4 atomes de carbone et/ou par un ou deux atomes d'halogène,
- un groupe phényle,
- un groupe phényle substitué par un à trois groupes alkyles ayant 1 à 4 atomes de carbone et/ou par un ou deux atomes d'halogène,
- R₂ représente :
- un groupe alkyle, linéaire ou ramifié, ayant 1 à 6 atomes de carbone,
- un groupe cycloalkyle ayant 5 à 6 atomes de carbone,
- un groupe cycloalkyle, ayant 5 ou 6 atomes de carbone substitué par un à trois groupes alkyle ayant 1 à 4 atomes de carbone et/ou par un ou deux atomes d'halogène,
- un groupe phényle,
- un groupe phényle substitué par un à trois groupes alkyle ayant 1 à 4 atomes de carbone et/ou par un ou deux atomes d'halogène,
- R₁ et R₂ peuvent former ensemble un groupe alkylène ayant 2 à 6 atomes de carbone.

6. Procédé selon la revendication 5 **caractérisé par le fait que** le carbonate organique répond à la formule (II) dans laquelle les groupes R₁, R₂ représentent un groupe alkyle ayant de 1 à 4 atomes de carbone, de préférence méthyle, éthyle, isopropyle ; un groupe cyclohexyle ; un groupe phényle : un groupe phényle substitué en ortho et ortho' par un atome d'halogène de préférence chlore ou brome ou par un groupe alkyle linéaire ou ramifié ayant de 1 à 4 atomes de carbone ou les groupes R₁ et R₂ forment un groupe alkylène, de préférence un groupe d'éthylène ou de propylène.

7. Procédé selon l'un des revendications 5 et 6 **caractérisé par le fait que** le carbonate organique est le carbonate de diméthyle, le carbonate de diéthyle, le carbonate de diisopropyle, le carbonate de phényle et de tertiobutyle, le carbonate d'éthylène, le carbonate de propylène.

8. Procédé selon la revendication 7 **caractérisé par le fait que** le carbonate organique est le carbonate de diméthyle ou de diéthyle.

9. Procédé selon l'un des revendications 1 à 8 **caractérisé par le fait que** la quantité de carbonate organique mise en oeuvre exprimée par rapport à l'acide fluoroalcanesulfinique est telle que le rapport entre le nombre de moles de carbonate organique et le nombre de moles d'acide fluoroalcanesulfinique varie entre 1 et 2 et se situe de préférence entre 1 et 1,2.

10. Procédé selon l'un des revendications 1 à 9 **caractérisé par le fait que** la réaction est conduite dans des conditions anhydres.

11. Procédé selon l'un des revendications 1 à 10 **caractérisé par le fait que** la température de la réaction est choisie entre 0 et 100°C, de préférence entre 60 et 95°C.

12. Procédé selon l'un des revendications 1 à 11 **caractérisé par le fait que** la réaction est conduite sous pression atmosphérique et sous atmosphère de gaz inerte.

13. Procédé selon l'un des revendications 1 à 12 **caractérisé par le fait que** le dioxyde de carbone est éliminé au fur et à mesure de sa formation.

14. Procédé selon l'un des revendications 1 à 13 **caractérisé par le fait que** l'ester de l'acide fluoroalcanesulfinique est récupéré à partir du milieu obtenu comprenant l'ester de l'acide fluoroalcanesulfinique et l'alcool, par distillation ou bien par cristallisation.

## Claims

1. Process for preparing an ester of a fluoroalkanesulfinic acid, **characterized in that** it comprises reacting a fluoroalkanesulfinic acid with an organic carbonate, resulting in the formation of a fluoroalkanesulfinic acid ester and carbon dioxide which is removed during the reaction.

2. Process according to Claim 1, **characterized in that** the fluoroalkanesulfinic acid corresponds to the following formula: 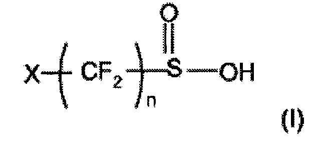 in which formula:
- X represents a hydrogen atom or a fluorine atom,
- n represents a number between 1 and 8.

3. Process according to Claim 2, **characterized in that** the fluoroalkanesulfinic acid is difluoromethanesulfinic acid, a perfluoroalkanesulfinic acid, preferably trifluoromethanesulfinic acid, perfluorobutanesulfinic acid or perfluorooctanesulfinic acid.

4. Process according to Claim 2, **characterized in that** the fluoroalkanesulfinic acid is trifluoromethanesulfinic acid.

5. Process according to one of Claims 1 to 4, **characterized in that** the organic carbonate corresponds to the following general formula:
R₁-O-CO-O-R₂ (II)
in which formula:
- R₁ represents:
- a linear or branched alkyl group containing 1 to 6 carbon atoms,
- a cycloalkyl group containing 5 or 6 carbon atoms,
- a cycloalkyl group containing 5 or 6 carbon atoms which is substituted with one to three alkyl groups containing 1 to 4 carbon atoms and/or with one or two halogen atoms,
- a phenyl group,
- a phenyl group substituted with one to three alkyl groups containing 1 to 4 carbon atoms and/or with one or two halogen atoms,
- R₂ represents:
- a linear or branched alkyl group containing 1 to 6 carbon atoms,
- a cycloalkyl group containing 5 or 6 carbon atoms,
- a cycloalkyl group containing 5 or 6 carbon atoms which is substituted with one to three alkyl groups containing 1 to 4 carbon atoms and/or with one or two halogen atoms,
- a phenyl group,
- a phenyl group substituted with one to three alkyl groups containing 1 to 4 carbon atoms and/or with one or two halogen atoms,
- R₁ and R₂ can together form an alkylene group containing 2 to 6 carbon atoms.

6. Process according to Claim 5, **characterized in that** the organic carbonate corresponds to formula (II) in which the R₁ and R₂ groups represent an alkyl group containing from 1 to 4 carbon atoms, preferably methyl, ethyl, isopropyl; a cyclohexyl group; a phenyl group; or a phenyl group ortho- and ortho' -substituted with a halogen atom, preferably chlorine or bromine, or with a linear or branched alkyl group containing from 1 to 4 carbon atoms, or the R₁ and R₂ groups form an alkylene group, preferably an ethylene or propylene group.

7. Process according to either of Claims 5 and 6, **characterized in that** the organic carbonate is dimethyl carbonate, diethyl carbonate, diisopropyl carbonate, tert-butyl phenyl carbonate, ethylene carbonate or propylene carbonate.

8. Process according to Claim 7, **characterized in that** the organic carbonate is dimethyl carbonate or diethyl carbonate.

9. Process according to one of Claims 1 to 8, **characterized in that** the amount of organic carbonate used, expressed relative to the fluoroalkanesulfinic acid, is such that the ratio of the number of moles of organic carbonate to the number of moles of fluoroalkanesulfinic acid ranges between 1 and 2, and is preferably between 1 and 1.2.

10. Process according to one of Claims 1 to 9, **characterized in that** the reaction is carried out under anhydrous conditions.

11. Process according to one of Claims 1 to 10, **characterized in that** the reaction temperature is selected between 0 and 100°C, preferably between 60 and 95°C.

12. Process according to one of Claims 1 to 11, **characterized in that** the reaction is carried out under atmospheric pressure and under the atmosphere of an inert gas.

13. Process according to one of Claims 1 to 12, **characterized in that** the carbon dioxide is removed as it forms.

14. Process according to one of Claims 1 to 13, **characterized in that** the fluoroalkanesulfinic acid ester is recovered from the medium obtained comprising the fluoroalkanesulfinic acid ester and the alcohol, by distillation or else by crystallization.

## Patentansprüche

1. Verfahren zur Herstellung eines Esters einer Fluoralkansulfinsäure, **dadurch gekennzeichnet, dass** man eine Fluoralkansulfinsäure mit einem organischen Carbonat umsetzt, was zur Bildung eines Fluoralkansulfinsäureesters und von Kohlendioxid, das während der Umsetzung entfernt wird, führt.

2. Verfahren nach Anspruch 1, **dadurch gekennzeichnet, dass** die Fluroalkansulfinsäure der folgenden Formel entspricht: 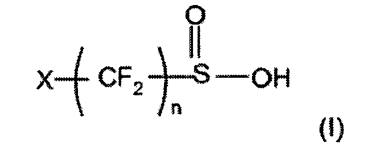 worin:
- X für ein Wasserstoffatom oder ein Fluoratom steht;
- n für eine Zahl zwischen 1 und 8 steht.

3. Verfahren nach Anspruch 2, **dadurch gekennzeichnet, dass** es sich bei der Fluoralkansulfinsäure um Difluormethansulfinsäure oder eine Perfluoralkansulfinsäure, vorzugsweise Trifluormethansulfinsäure, Perfluorbutansulfinsäure oder Perfluoroctansulfinsäure, handelt.

4. Verfahren nach Anspruch 2, **dadurch gekennzeichnet, dass** es sich bei der Fluoralkansulfinsäure um Trifluormethansulfinsäure handelt.

5. Verfahren nach einem der Ansprüche 1 bis 4, **dadurch gekennzeichnet, dass** das organische Carbonat der folgenden allgemeinen Formel entspricht:
R₁-O-CO-C-R₂ (II)
worin:
- R₁ für:
- eine lineare oder verzweigte Alkylgruppe mit 1 bis 6 Kohlenstoffatomen,
- eine Cycloalkylgruppe mit 5 bis 6 Kohlenstoffatomen,
- eine Cycloalkylgruppe mit 5 bis 6 Kohlenstoffatomen, die durch eine bis drei Alkylgruppen mit 1 bis 4 Kohlenstoffatomen und/oder durch ein oder zwei Halogenatome substituiert ist,
- eine Phenylgruppe,
- eine Phenylgruppe, die durch eine bis drei Alkylgruppen mit 1 bis 4 Kohlenstoffatomen und/oder durch ein oder zwei Halogenatome substituiert ist,
steht;
- R₂ für:
- eine lineare oder verzweigte Alkylgruppe mit 1 bis 6 Kohlenstoffatomen,
- eine Cycloalkylgruppe mit 5 bis 6 Kohlenstoffatomen,
- eine Cycloalkylgruppe mit 5 bis 6 Kohlenstoffatomen, die durch eine bis drei Alkylgruppen mit 1 bis 4 Kohlenstoffatomen und/oder durch ein oder zwei Halogenatome substituiert ist,
- eine Phenylgruppe,
- eine Phenylgruppe, die durch eine bis drei Alkylgruppen mit 1 bis 4 Kohlenstoffatomen und/oder durch ein oder zwei Halogenatome substituiert ist,
steht;
- R₁ und R₂ zusammen Alkylengruppe mit 2 bis 6 Kohlenstoffatomen bilden können.

6. Verfahren nach Anspruch 5, **dadurch gekennzeichnet, dass** das organische Carbonat der Formel (II) entspricht, worin die Gruppen R₁ und R₂ für eine Alkylgruppe mit 1 bis 4 Kohlenstoffatomen, vorzugsweise Methyl, Ethyl oder Isopropyl; eine Cyclohexylgruppe; eine Phenylgruppe; eine Phenylgruppe, die in ortho- und ortho'-Stellung durch ein Halogenatom, vorzugsweise Chlor oder Brom, oder durch eine lineare oder verzweigte Alkylgruppe mit 1 bis 4 Kohlenstoffatomen substituiert ist; stehen oder die Gruppen R₁ und R₂ eine Alkylengruppe, vorzugsweise eine Ehtylen- oder Propylengruppe, bilden.

7. Verfahren nach einem der Ansprüche 5 und 6, **dadurch gekennzeichnet, dass** es sich bei dem organischen Carbonat um Dimethylcarbonat, Diethylcarbonat, Diisopropylcarbonat, Phenyl-tert.-butyl-carbonat, Ethylencarbonat oder Propylencarbonat handelt.

8. Verfahren nach Anspruch 7, **dadurch gekennzeichnet, dass** es sich bei dem organischen Carbonat um Dimethylcarbonat oder Diethylcarbonat handelt.

9. Verfahren nach einem der Ansprüche 1 bis 8, **dadurch gekennzeichnet, dass** das organische Carbonat in einer solchen Menge, ausgedrückt im Verhältnis zu der Fluoralkansulfinsäure, eingesetzt wird, dass das Verhältnis zwischen der Zahl der Mole von organischem Carbonat und der Zahl der Mole von Fluoralkansulfinsäure zwischen 1 und 2 variiert und vorzugsweise zwischen 1 und 1,2 liegt.

10. Verfahren nach einem der Ansprüche 1 bis 9, **dadurch gekennzeichnet, dass** man die Umsetzung unter wasserfreien Bedingungen durchführt.

11. Verfahren nach einem der Ansprüche 1 bis 10, **dadurch gekennzeichnet, dass** man die Reaktionstemperatur zwischen 0 und 100°C, vorzugsweise zwischen 60 und 95°C, wählt.

12. Verfahren nach einem der Ansprüche 1 bis 11, **dadurch gekennzeichnet, dass** man die Umsetzung unter Normaldruck und unter Inertgasatmosphäre durchführt.

13. Verfahren nach einem der Ansprüche 1 bis 12, **dadurch gekennzeichnet, dass** man das Kohlendioxid so schnell entfernt, wie es sich bildet.

14. Verfahren nach einem der Ansprüche 1 bis 13, **dadurch gekennzeichnet, dass** man den Fluoralkansulfinsäureester aus dem den Fluoralkansulfinsäureester und den Alkohol enthaltenden Reaktionsmedium durch Destillation oder auch durch Kristallisation gewinnt.
